# EUROPEAN PATENT SPECIFICATION

(11) **EP 2 736 496 B1**
(45) Date of publication and mention of the grant of the patent: **05.09.2018**
(21) Application number: 12751445.3
(22) Date of filing: 20.07.2012
(51) Int. Cl.: A61K 9/20, A61K 9/28, A61K 9/48, A61K 31/137

(54) **PHARMACEUTICAL COMPOSITION CONTAINING AN ANTIMUSCARINIC AGENT AND METHOD FOR THE PREPARATION THEREOF**
PHARMAZEUTISCHE ZUSAMMENSETZUNG MIT EINEM ANTIMUSKARINISCHEN WIRKSTOFF UND HERSTELLUNGSVERFAHREN DAFÜR
COMPOSITION PHARMACEUTIQUE CONTENANT UN AGENT ANTIMUSCARINIQUE ET SON PROCÉDÉ DE PRÉPARATION

(30) Priority: 27.07.2011 GR 20110100444
(43) Date of publication of application: 04.06.2014
(73) Proprietor: Pharmathen S.A., 15351 Pallini Attikis (GR)
(72) Inventor: KARAVAS, Evangelos, 153 51 Pallini Attikis (GR); KOUTRIS, Efthimios, 153 51 Pallini Attikis (GR); KOUTRI, Ioanna, 153 51 Pallini Attikis (GR); SAMARA, Vicky, 153 51 Pallini Attikis (GR); BIKIARIS, Dimitrios, 54351 Thessaloniki (GR); ILIOPOULOU, Athina, 153 51 Pallini Attikis (GR)
(86) International application number: PCT/EP2012/003076
(87) International publication number: WO 2013/013798

(56) References cited:
- EP-A1- 1 731 142
- EP-A1- 1 810 668
- WO-A1-2005/105036
- WO-A1-2010/096820
- US-A1- 2009 214 665

## Description

### TECHNICAL FIELD OF THE INVENTION

The present invention relates to a modified release dosage form and in particular to a pharmaceutical composition for oral administration comprising a therapeutically effective quantity of Tolterodine or pharmaceutical acceptable salt thereof and a method for manufacturing such composition.

### BACKGROUND OF THE INVENTION

Urinary incontinence is the involuntary excretion of urine from one's body. It is assumed that unstable or overactive bladder is caused by uncontrolled contractions of the bundles of smooth muscle fibers, forming the muscular coat of the urinary bladder, during its filling phase. The pharmacological treatment in such cases is the administration of muscarinic receptor antagonists such as Oxybutynin and Tolterodine.

Tolterodine is the (R)-N, N-diisopropyl-3-(2-hydroxy-5-methylphenyl)-3-phenylpropylamine and is an antimuscarinic drug that is used to treat urinary incontinence and other symptoms of unstable or overactive urinary bladder and has a water solubility of 12mg/ml. It acts on M2 and M3 subtypes of muscarinic receptors whereas most antimuscarinic agents only act on M3 receptors. Tolterodine targets the bladder more than other areas of the body thus lower dose needs to be given daily (due to efficient targeting) and so causing fewer side effects.

Nevertheless, commercially sold immediate release compositions of Tolterodine are associated with side effects such as dry mouth, dyspepsia, headache due to high concentration of the drug in a short period of time. In order to overcome this problem extended release f compositions have been developed. The reference extended release product of Tolterodine is in the form of a hard gelatine capsule containing beads, consisting of an inert core (sugar spheres containing sucrose and maize starch) covered with a drug layer and a water soluble polymer (hypromellose), and further coated with a controlled release polymer layer (ethylcellulose, medium chain triglycerides and oleic acid).

Extended release dosage forms are widely used for the administration of a variety of drugs since they maintain substantially constant blood levels and avoid the fluctuations associated with the immediate release compositions. Further, the extended release compositions provide many advantages over the immediate release dosage forms, such as increased patients compliance, improved delivery efficiency, decreased total drug requirement and minimization or even elimination of local or systemic side effects.

Various methods are already known for the industrial preparation of extended release oral dosage forms comprising an antimuscarinic agent, and in particular Tolterodine or a pharmaceutical acceptable salt thereof as an active ingredient due to its useful therapeutic properties. However, the prior art has encountered substantial difficulties in the production of oral solid compositions having a desirable release rate for once a day administration but yet a cost effective manufacturing process.

EP 1 128 819 discloses a composition containing extended release beads comprising a core unit of a substantially water soluble or water swellable inert material, a first layer on the core of a substantially water insoluble polymer, a second layer over the first that contains an active ingredient and a third layer of polymer on the second layer effective for controlled release of the active ingredient, wherein the first layer is adapted to control water penetration into the core. This process is very complex, not cost effective and time consuming.

WO 2007/122015 discloses a controlled release pharmaceutical formulation comprising a bead of a sugar core, a sealcoat layer surrounding the core comprising HPMC, a drug layer comprising Tolterodine and a hydrophilic polymer and a controlled release layer comprising a water insoluble polymer and a pore forming component material.

WO 2007/029087 discloses a controlled release multiple unit pharmaceutical composition comprising an inert core comprising EC and optionally one or more water soluble or water swellable excipients, an active layer comprising one or more active ingredients and one or more hydrophilic polymers and a polymeric layer for controlling or modifying the release.

US 2009/214665 discloses a modified release composition comprising an extruded spheronized core, a rapidly dissolving coating, a controlled release coating and a delayed release coating.

Although each of the above patents represents an attempt to achieve a desirable release rate for once a day administration with an extended release pharmaceutical composition comprising an antimuscarinic agent, there is still exist a need to provide a stable modified release composition with low production costs and without producing unwanted pharmaceutical side effects.

### SUMMARY OF THE INVENTION

It is, therefore, an object of the present invention to provide an improved modified release solid dosage composition for oral administration containing Tolterodine or pharmaceutical acceptable salt thereof as an active ingredient according to claim 1, which overcomes the deficiencies of the prior art.

It is another object of the present invention to provide a stable modified release solid pharmaceutical dosage composition for oral administration containing Tolterodine or pharmaceutical acceptable salt thereof as an active ingredient, which is bioavailable and effective with sufficient self-life, good pharmacotechnical properties, enhancing patient compliance and reducing possible side effects.

Moreover, another aspect of the present invention is to provide a modified release solid dosage composition for oral administration containing Tolterodine or pharmaceutical acceptable salt thereof as an active ingredient, which can be prepared in dosage forms of different strength by proportionally adjusting the quantities of the excipients and the active ingredient, thereby providing a pharmacotechnical linearity, without affecting the dissolution profile and bioavailability of the active ingredient.

A further aspect of the present invention is to provide a method for the preparation of a modified release solid dosage formulation for oral administration containing Tolterodine or pharmaceutical acceptable salt thereof as an active ingredient according to claim 4, thereby enhancing the release rate of the active ingredient and being stable over a long period of time and improving the pharmacotechnical characteristics of the composition.

In accordance with the above objects of the present invention, a modified release pharmaceutical composition for oral administration is provided comprising Tolterodine or pharmaceutical acceptable salt thereof as an active ingredient, in a matrix tablet coated with a release modifying coating system.

According to another embodiment of the present invention, a modified release pharmaceutical composition for oral administration is provided comprising a hard gelatine capsule with a therapeutically effective number of mini tablets, said mini tablets comprising Tolterodine or pharmaceutical acceptable salt thereof as an active ingredient, incorporated in a matrix of a water soluble and a water insoluble polymer and a release modifying coating system, as a way to improve the release characteristic of the active ingredient.

According to another embodiment of the present invention, a process for the preparation of a modified release solid dosage forms for oral administration according to claim 4 containing Tolterodine or pharmaceutical acceptable salt thereof as an active ingredient embedded in a matrix core, said core being coated with a first coating layer of a hydrophobic polymer and a second coating layer of a gastric pH resistance polymer in order to improve the release characteristic of the active ingredient is provided, which comprises:
- dissolving the total quantity of a wetting agent in water;
- forming a homogenous mixture by mixing the total quantity of said active ingredient with the total quantity of a water insoluble polymer;
- kneading the above mixture with the wetting agent solution;
- adding to the formed solution the total quantity of a water soluble polymer and the total quantity of at least one optional excipient such as a diluent, a binder, a disintegrant, a glidant, a lubricant and wet granulating until uniform;
- drying the wetted mass;
- sieving the dried mass and adding to the sieved mixture the total quantities of at least one optional excipient such as a binder, a diluent, a disintegrant, a lubricant and/or a glidant and mixing until uniform;
- formulating the resulting mixture in a solid dosage form by compressing it into a desired tablet or mini tablet form;
- applying on the solid dosage form a first rate controlling coating comprising a hydrophobic agent;
- applying on top of the first coating a second coating comprising an agent that is not soluble in gastric pH, and
- filling the mini tablets into hard gelatine capsules

### DETAILED DESCRIPTION OF THE INVENTION

For the purposes of the present invention, a pharmaceutical composition comprising Tolterodine or pharmaceutical acceptable salt thereof is considered to be "stable" if said ingredient degrades less or more slowly than it does on its own and/or in known pharmaceutical compositions.

The active ingredient contained in a dosage form is "bioavailable", if when administered in a dosage form is released from the dosage form, absorbed and reaches, at least the same, concentration levels in plasma as any of the marketed products containing the same quantity of the same active ingredient and intended for the same use.

An excipient is considered to be "incompatible" with an active ingredient (an antimuscarinic agent i.e. Tolterodine or pharmaceutical acceptable salt thereof) if it promotes the degradation of said active ingredient, that is to say, if said active ingredient degrades more or faster in the presence of said excipient when compared with the degradation of said active ingredient on its own. The terms "incompatibility", "compatible" and "compatibility" are defined accordingly.

The term "pharmaceutically acceptable" refers to a form that is acceptable to the patient from a pharmacological or toxicological point of view, including acceptable composition, stability, and bioavailability.

As already mentioned the main object of the present invention is to provide a modified release composition of Tolterodine or pharmaceutical acceptable salt thereof that is simple to manufacture, bioavailable, cost effective, stable, possesses good pharmacotechnical properties and linearity.

A well-known problem when trying to manufacture modified release dosage forms, of water-soluble active ingredients, such as Tolterodine, is the rapid initial drug release. It has been surprisingly found that this problem is solved by the present invention by using a matrix core and a release modifying coating system in order to control the release rate of the active substance from the pharmaceutical composition of Tolterodine or pharmaceutical acceptable salt thereof.

The core matrix system comprises a mixture of a water insoluble and water soluble polymer together with a wetting agent and other optional pharmaceutically acceptable excipients.

The modifying release coating system comprises two coating layers on the matrix core. The first coating layer comprises a hydrophobic polymer and the second coating layer comprises a gastric pH resistance polymer.

The release mechanism of the composition of the present invention, involves the synergetic effect of the matrix core, the first coating layer and second coating layer. The coating layer is applied in small amounts. The first coating layer is applied around the matrix core and does not add more than 5% by weight to the uncoated core, while the second coating layer is applied on top of the first coating and does not add more than 5% by weight to the core with the first coating.

The present invention provides solid dosage forms that exhibit linearity between the strength of the drug formulation and the total mass of the formulation, wherein the drug delivery from the matrix tablets, is highly affected by the geometrical characteristics of the tablet-matrix.

This phenomenon prevents the achievement of linearity between the different strengths of the drug product and the total weight of the dosage form. As the strength gets bigger and the size of the matrix increases the dissolution rate is delayed, so as the dosage forms that refer to different strengths of the same drug product do not exhibit the same dissolution profile.

Linearity between the strength and the composition of a dosage form without the release characteristics being influenced is highly desired in the pharmaceutical industry for manufacturing, pharmacokinetic and economical reasons.

According to the present invention, the modified release pharmaceutical composition of Tolterodine or pharmaceutical acceptable salt thereof may be in various forms. The preferred solid forms are tablets, capsules and caplets. More preferred are mini tablets filled in a hard gelatin capsule. The mini tablets may have a length of from about 1mm to 7mm and width of from about 1mm to 7mm but preferably are round biconvex minitablets of about 4mm diameter.

Tableting was the chosen production method because it is faster, easier, adds fewer steps to the process and is the most economical. Further, the tableting method ensures a high production yield, contrary to the manufacture of pellets or beads where the loss of production output is usually much higher.

The use of mini-tablets is beneficial because pharmacotechnical linearity between the strength and the composition is achieved easily by incorporating one or more of the mini-tablets in the capsule.

Another embodiment of the present invention is the use of the wet granulation process for the preparation of a modified release solid dosage forms for oral administration, such as mini tablets to be filled in hard gelatine capsules, comprising Tolterodine or pharmaceutical acceptable salt thereof as an active ingredient embedded in a matrix core, said core being coated with a first coating layer of a hydrophobic polymer and a second coating layer of a gastric pH resistance polymer in order to improve the release characteristic of the active ingredient. Said wet granulation process comprises:
- dissolving the total quantity of a wetting agent in water;
- forming a homogenous mixture by mixing the total quantity of said active ingredient with the total quantity of a water insoluble polymer;
- kneading the above mixture with the wetting agent solution;
- adding to the formed solution the total quantity of a water soluble polymer and the total quantity of at least one optional excipient such as a diluent, a binder, a disintegrant, a glidant, a lubricant and wet granulating until uniform;
- drying the wetted mass;
- sieving the dried mass and adding to the sieved mixture the total quantities of at least one optional excipient such as a binder, a diluent, a disintegrant, a lubricant and/or a glidant and mixing until uniform;
- formulating the resulting mixture in a solid dosage form by compressing it into a desired tablet or mini tablet form;
- applying on the solid dosage form a first rate controlling coating comprising a hydrophobic agent;
- applying on top of the first coating a second coating comprising an agent that is not soluble in gastric pH, and filling the mini tablets into hard gelatine capsules

The manufacturing process for the preparation of the composition according to the present invention is simpler and inexpensive in comparison to other method.

The pharmaceutical composition according to the present invention is characterized by excellent pharmacotechnical properties, such as homogeneity, flowability and compressibility. Namely, the pure pharmaceutical substance Tolterodine tartrate showed limited flowability and compressibility with a mean Carr's Index of 38, 7%. However, when a lubricant is incorporated into the composition, the Carr Index observed was 23,2%, which indicates an improvement of the flow properties of Tolterodine composition.

The pharmaceutical compositions of the present invention may also contain one or more additional pharmaceutically acceptable ingredients selected from a wide variety of excipients. According to the desired properties, any number of ingredients may be selected, alone or in combination, based upon their known uses in preparation of solid dosage form compositions.

Such ingredients include diluents, binders, compression aids, disintegrants, surfactants, antioxidants, glidants, lubricants, flavours, water scavengers, colorants, sweetener, coating agents and preservatives.

Diluents may be, for example, calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin, maltitol.

Binders may be, for example, acacia mucilage, alginic acid, carbomer, carboxymethylcellulose calcium, carboxymethylcellulose sodium, microcrystalline cellulose, powdered cellulose, ethyl cellulose, gelatin, liquid glucose, guar gum, hydroxyethyl cellulose, hydroxypropyl cellulose, hydroxypropylmethyl cellulose, maltodextrin, methylcellulose, polydextrose, polyethylene oxide, povidone, sodium alginate, starch paste, pregelatinized starch and sucrose.

Disintegrants may be, for example, alginic acid, carbon dioxide, carboxymethylcellulose calcium, carboxymethylcellulose sodium, microcrystalline cellulose, powdered cellulose, crosscarmelose sodium, crospovidone, sodium docusate, guar gum, hydroxypropyl cellulose, methylcellulose, polacrilin potassium, poloxamer, povidone, sodium alginate, sodium glycine carbonate, sodium lauryl sulfate, sodium starch glycolate, starch, pregelatinized starch.

Surfactants may be, polyoxyethylene-polyoxypropylene co-polymers and block co-polymers, commercially available as Pluronic® or Poloxamer®, ethoxylated cholesterins, commercially available as Solulan®, vitamin derivatives, e.g. vitamin E derivatives such as tocopherol polyethylene glycol succinate (TPGS), sodium dodecylsulfate or sodium laurylsulfate; a bile acid or salt thereof, for example cholic acid, glycolic acid or a salt.

Glidants may be, for example, calcium silicate, powdered cellulose, starch, talc, colloidal silicon dioxide.

Lubricants such as polyethylene glycol 4000, polyethylene glycol 6000, sodium lauryl sulfate, starch, talc.

One or more suitable water soluble polymers may be selected to be used in the invention, which include cellulose derivatives (e.g. hydroxypropylmethylcellulose, hydroxypropylcellulose, methylcellulose, etc.), alginic acid, propylene glycol, pectin, low methoxy pectin, guar gum, gum arabic, carrageenan, xanthan gum,synthetic water-soluble polymer (e.g. polyvinylpyrrolidone, etc.).

One or more suitable water insoluble polymers may be selected to be used in the invention, which include cellulose derivatives (e.g. ethylcellulose), polyvinyl acetate (Kollidon SR), neutral copolymers based on ethyl acrylate and methylmethacrylate, copolymers of acrylic and methacrylic acid esters with quaternary ammonium groups, such as Eudragit NE, RS or RS30D, RL or RL30D.

The hydrophobic polymer of the first coating layer applied on the matrix core, may be for example ethylcellulose, polyvinyl acetate, ammoniomethacrylates, methyl methacrylate, cellulose acetate, etc. The second coating layer that is resistant to gastric pH values comprises Kollicoat MAE 30DP and 1,2-propylene glycol and is less than 5% weight of the total weight of the coated, with the first coating, matrix core. The coating may also include a plasticizer selected from triacetin, triethylcitrate, polyethylene glycol, acetyltriethylcitrate, miglyol, propylene glycol, diethylphthalate, meglumine, etc.

Moreover, any excipient may optionally be added to the above composition, provided that they are compatible with the active ingredient, in order to overcome problems associated with the unfavorable pharmacotechnical characteristics of these substances, and in order to increase the stability of the drug and the self-life of the pharmaceutical product, and provide a product exhibiting excellent bioavailability.

The following examples illustrate preferred embodiments in accordance with the present invention:

### EXAMPLES

### Example 1

**Table 1: Compositions 1.1, 1.2 and 1.3 of Tolterodine**

| **Composition** | **1.1** | **1.2** | **1.3** |
|---|---|---|---|
| **Ingredients** | **% by weight** | | |
| Tolterodine Tartrate | 2.50 | 2.50 | 2.50 |
| Microcellac | 36.00 | 46.00 | 56.00 |
| Kollidon SR | 10.00 | 10.00 | 10.00 |
| Methocel K100M | 50.00 | 40.00 | 30.00 |
| Sodium Docusate | 1.00 | 1.00 | 1.00 |
| Mg Stearate | 0.50 | 0.50 | 0.50 |
| **Total** | 100.00 | 100.00 | 100.00 |

Minitablets of the above compositions (composition 1.1, composition 1.2 and composition 1.3) were prepared according to the following manufacturing process: Sodium Docusate was dissolved in water. Tolterodine Tartrate was mixed with Kollidon SR to form a homogenous mixture. The above mixture was kneaded with the solution of Sodium Docusate. Microcellac and Methocel K100M were added and wet granulated. The granular mass was dried. Finally Mg Stearate was added to the dried granule and mixed until complete homogeneity. The resulting granule was compressed into minitablets and filled into capsules.

The three compositions as shown in Table 1 having different percentage of Methocel K100M were subjected to a dissolution test in a medium of pH 1.2 up to the first two hours with changement of the medium to buffer pH 6.8 for the rest of the time. The dissolution profiles have been compared with the dissolution profile of the reference product, which comprises Tolterodine L-tartrate and the following excipients: sugar spheres (containing sucrose and maize starch), Surelease E-7-9010 clear, ethylcellulose, medium chain triglycerides, oleic acid and hypromellose.

**Table 2: Dissolution profiles of compositions 1.1, 1.2 and 1.3 according to Example 1**

| **Time(h)** | **% Dissolved *(buffer pH 1.2 to 6.8)*** | | | |
|---|---|---|---|---|
| | **1.1** | **1.2** | **1.3** | **Reference** |
| 1 | 15.5 | 18.5 | 20.8 | 13.7 |
| 2 | 26.3 | 30.9 | 33.1 | 22.1 |
| 3 | 30.8 | 38.3 | 39.5 | 26.8 |
| 4 | 36.7 | 43.4 | 45.2 | 34.2 |
| 5 | 41.9 | 47.8 | 51.6 | 41.4 |
| 7 | 51.9 | 56.0 | 62.6 | 61.2 |
| 9 | 59.0 | 62.0 | 68.7 | 78.6 |
| 12 | 69.1 | 70.6 | 76.5 | 89.9 |
| 15 | 77.2 | 76.9 | 83.2 | 92.9 |
| 18 | 81.3 | 82.8 | 88.6 | 96.8 |
| 20 | 86.2 | 86.3 | 92.9 | 99.4 |

The results obtained, as expected, presented a higher release rate for composition 1.3 having the smaller percentage of Methocel K100M. Composition 1.3 resembles better the dissolution profile of the reference product after the first few hours, compared to that of compositions 1.1 and 1.2. Due to Tolterodine's water solubility the release at the beginning is much higher than the desirable.

### Example 2: Compositions 1.3.1, 1.3.2, 1.3.3 and 1.3.4

Minitablets of composition 1.3 of example 1 were coated with a layer consisting of ethylcellulose N10 and Citrofol (20% on ethylcellulose) in order to suppress the initial release of Tolterodine. Mini tablets were removed from the coating machine in various time periods.

**Table 3: Dissolution profiles of compositions 1.3.1, 1.3.2, 1.3.3 and 1.3.4**

| **Time(h)** | **% Dissolved *(buffer pH 1.2 to 6.8)*** | | | |
|---|---|---|---|---|
| | **1.3.1** | **1.3.2** | **1.3.3** | **1.3.4** |
| 1 | 20.2 | 18.7 | 10.6 | 1.4 |
| 2 | 35.4 | 35.4 | 24.9 | 1.4 |
| 3 | 44.6 | 41.4 | 34.6 | 2.7 |
| 4 | 51.4 | 47.6 | 39.9 | 4.4 |
| 5 | 57.5 | 53.6 | 45.6 | 8.1 |
| 7 | 68.4 | 63.2 | 54.3 | 18.9 |
| 9 | 76.8 | 71.8 | 62.3 | 31.3 |
| 12 | 86.6 | 81.7 | 73.1 | 47.8 |
| 15 | 94.0 | 90.5 | 82.1 | 61.0 |
| 18 | 100.0 | 98.2 | 89.8 | 70.6 |
| 20 | 102.8 | 102.9 | 94.2 | 76.1 |

When the mini tablets remain longer in the coating machine, the volume of the applied coating and thus, the weight gained of the composition are higher.

Four different compositions 1.3.1, 1.3.2, 1.3.3 and 1.3.4 with increased coating weight, namely 1.0%, 1.5%, 2% and 5% weight respectively to the weight of uncoated tablets of composition 1.3, were tested.

Composition 1.3.2 as shown in Table 3 below resembles better the dissolution profile of the reference product.

### Example 3: Preferred compositions 1.3.2.1, 1.3.2.2 and 1.3.2.3 according to the present invention

Minitablets of composition 1.3.2 of example 2 above, having a weight gain of 1.5% on the initial weight of uncoated tablets are further coated with a second layer consisting of Kollicoat MAE 30DP and 1, 2-propylene glycol, in order to further suppress the release of the Tolterodine.

The mini tablets are removed from the coating machine in various time periods according to the weight gain during the coating process.

Three different compositions 1.3.2.1, 1.3.2.2 and 1.3.2.3 with increased coating weight, namely 1.0%, 2.0% and 3.0% weight respectively added to the weight of coated tablets of composition 1.3.2, were tested.

**Table 4: Dissolution profiles of compositions 1.3.2.1, 1.3.2.2 and 1.3.2.3**

| **Time(h)** | **% Dissolved *(buffer pH 1.2 to 6.8)*** | | |
|---|---|---|---|
| | **1.3.2.1** | **1.3.2.2** | **1.3.2.3** |
| 1 | 9.8 | 5.8 | 1.0 |
| 2 | 24.9 | 14.9 | 3.4 |
| 3 | 33.9 | 24.9 | 13.7 |
| 4 | 40.4 | 34.0 | 24.8 |
| 5 | 45.7 | 42.2 | 34.0 |
| 7 | 53.8 | 53.9 | 45.9 |
| 9 | 60.9 | 62.8 | 54.8 |
| 12 | 70.2 | 74.5 | 64.8 |
| 15 | 77.8 | 82.1 | 74.0 |
| 18 | 84.9 | 88.4 | 82.0 |
| 20 | 88.8 | 91.1 | 87.1 |

Composition 1.3.2.2 as can be seen in Table 4 resembles better the dissolution profile of the reference product. The possibility to prepare dosage forms of different strength by adjusting the number of minitablets incorporated in the capsule, thereby limiting the cost of production and minimizing the number, and consequently the cost, of clinical studies required for the approval of the product by the authorities is another advantage of the composition.

Another object of the present invention was to prepare a pharmaceutical composition that is stable for a long period of storage time. Therefore, composition 1.3.2.2 was exposed to normal (25°C±2°C/60%±5% RH) and accelerated (40°C±2°C/75%±5% RH) stability studies according to the current ICH guidelines. The stability results are shown below.

**Table 5: Stability results of composition 1.3.2 directly after preparation and after 3 months of storage in normal and accelerated conditions**

| **IMPURITIES** | **%** | | |
|---|---|---|---|
| | **0 Months** | **3 Months** | |
| | | **Normal** | **Accelerated** |
| Imp 1 | ND | ND | ND |
| Imp 4 | 0.09 | 0.03 | 0.03 |
| Unknown | 0.09 | 0.02 | 0.19 |
| Unknown | 0.03 | 0.02 | 0.02 |
| Unknown | 0.06 | 0.05 | 0.61 |
| Total | 0.27 | 0.12 | 0.85 |

The stability of the product as well as the simple and economic manufacturing process indicates the advantages of the present invention relative to the commonly used methods and excipients for the composition of Tolterodine.

## Claims

1. A modified release pharmaceutical composition of Tolterodine or pharmaceutical acceptable salt thereof, as the active substance, for oral administration consisting of a matrix core comprising the active substance with a mixture of a water insoluble, a water soluble polymer and a wetting agent, a first coating layer over the core which comprises ethylcellulose and is less than 5% weight to the uncoated core and a second coating layer over the first coating layer which comprises Kollicoat MAE 30DP and 1 ,2-propylene glycol and is less than 5% weight of the total weight of the coated, with the first coating, matrix core.

2. The pharmaceutical composition according to claim 1, wherein said composition is a solid dosage form such as a tablet, sachet or mini tablets in a capsule.

3. The pharmaceutical composition according to claims 1 or 2, wherein it further comprises additional binders, fillers, glidants or lubricants.

4. A process for the preparation of a modified release solid dosage form of Tolterodine or a pharmaceutical acceptable salt thereof, as the active substance, for oral administration consisting of a matrix core comprising the active substance with a mixture of a water insoluble, a water soluble polymer and a wetting agent, a first coating layer over the core which comprises ethylcellulose and is less than 5% weight to the uncoated core and a second coating layer over the first coating layer which comprises Kollicoat MAE 30DP and 1 ,2-propylene glycol and is less than 5% weight of the total weight of the coated, with the first coating, matrix core, which process comprises:
- dissolving the total quantity of the wetting agent in water;
- forming a homogenous mixture by mixing the total quantity of Tolterodine or a pharmaceutical acceptable salt thereof with the total quantity of the water insoluble polymer;
- kneading the above mixture with the wetting agent solution;
- adding to the formed solution the total quantity of the water soluble polymer and the total quantity of at least one optional excipient such as a diluent, a binder, a disintegrant, a glidant, a lubricant and wet granulating until uniformity is achieved;
- drying the wetted mass;
- sieving the dried mass and adding to the sieved mixture the total quantities of at least one optional excipient such as a binder, a diluent, a disintegrant, a lubricant and/or a glidant and mixing until uniformity is achieved;
- formulating the resulting mixture in a solid dosage form by compressing it into a desired tablet or mini tablet form;
- applying a first coating layer over the core which comprises ethylcellulose;
- applying a second coating layer over the first coating layer which comprises Kollicoat MAE 30DP and 1 ,2-propylene glycol, and
- in case of mini-tablets; filling the mini tablets into hard gelatine capsules.

## Patentansprüche

1. Pharmazeutische Zusammensetzung mit modifizierter Wirkstofffreisetzung aus Tolterodin oder einem pharmazeutisch verträglichen Salz davon als aktive Substanz zur oralen Verabreichung, die aus einem Matrixkern bestehend, die aktive Substanz mit einer Mischung aus einem wasserunlöslichen Polymer, einem wasserlöslichen Polymer und einem Benetzungsmittel umfassend, eine erste Beschichtung Schicht über dem Kern, die Ethylcellulose umfasst und weniger als 5 Gew .-% des unbeschichteten Kerns beträgt, und eine zweite Beschichtungsschicht über der ersten Beschichtungsschicht, der Koolicoat MAE 30DP und 1,2-Propylenglykol umfasst und weniger als 5 Gew .-% des Gesamtgewichts der beschichteten, mit der ersten Beschichtung, Matrixkern ausmacht.

2. Pharmazeutische Zusammensetzung nach Anspruch 1, wobei die Zusammensetzung eine feste Dosierungsform wie eine Tablette, Sachet oder Minitabletten in einer Kapsel ist.

3. Pharmazeutische Zusammensetzung nach den Ansprüchen 1 oder 2, **dadurch gekennzeichnet, dass** sie zusätzlich Bindemittel, Füllstoffe, Gleitmittel oder Schmiermittel enthält.

4. Ein Verfahren zur Herstellung einer modifizierten Dosierungsform mit modifizierter Wirkstofffreisetzung von Tolterodin oder eines pharmazeutisch verträglichen Salzes davon als aktive Substanz zur oralen Verabreichung, die aus einem Matrixkern bestehend, die aktive Substanz mit einer Mischung aus einem wasserunlöslichen Polymer, einem wasserlöslichen Polymer und ein Benetzungsmittel umfassend, eine erste Überzugsschicht über dem Kern, die Ethylcellulose und weniger als 5 Gew .-% zu dem unbeschichteten Kern umfasst, und eine zweite Überzugsschicht über der ersten Überzugsschicht, die Kollicoat MAE 30DP und 1,2-Propylenglykol umfasst und weniger als 5 Gew .-% des mit der ersten Beschichtung beschichteten Matrixkerns ist, wobei das Verfahren die folgenden Schritte umfasst:
- der Gesamtmenge des Benetzungsmittels in Wasser auflösen
- einer homogenen Mischung durch Mischen der Gesamtmenge von Tolterodin oder eines pharmazeutisch verträglichen Salzes davon mit der Gesamtmenge des wasserunlöslichen Polymers bilden;
- der obigen Mischung mit der Benetzungsmittellösung kneten;
- der Gesamtmenge des wasserlöslichen Polymers und der Gesamtmenge von mindestens einem optionalen Hilfsstoff wie einem Verdünnungsmittel, einem Bindemittel, einem Sprengmittel, einem Gleitmittel, einer Schmiertmittel zugeben und zu der gebildeten Lösung, bis Gleichförmigkeit erreicht ist feuchtgranulieren;
- der nassen Masse trocknen
- der getrockneten Masse sieben und der Gesamtmengen an mindestens einem optionalen Hilfsstoff wie einem Bindemittel, einem Verdünnungsmittel, einem Dispergiermittel, einem Schmiermittel und/oder ein Gleitmittel zu der gesiebten Mischung zugaben; und, bis Gleichförmigkeit erreicht ist mischen;
- der resultierenden Mischung in einer festen Dosierungsform durch Komprimieren derselben in eine gewünschte Tabletten- oder Minitablettenform formulieren;
- einer ersten Überzugsschicht auf den Kern, der Ethylcellulose umfasst aufbringen;
- einer zweiten Überzugsschicht auf die erste Überzugsschicht, die Kollicoat MAE 30DP und 1,2-Propylenglykol enthält aufbringen, und
- Bei Minitabletten die Minitabletten in Hartgelatinekapseln füllen.

## Revendications

1. Une composition pharmaceutique à libération modifiée de la Toltérodine ou un sel pharmaceutiquement acceptable de celle-ci, comme principe actif, pour administration orale consistant d'un noyau matriciel comprenant le principe actif avec un mélange d'un polymère insoluble dans l'eau, un polymère soluble dans l'eau et un agent mouillant, une première couche d'enrobage sur le noyau qui comprend l'éthylcellulose et qui est de moins de 5% en poids du poids du noyau sans enrobage et une seconde couche de revêtement (d'enrobage), au-dessus de la première qui comprend Kollicoat MAE 30DP et le 1,2-propylène glycol et qui est de moins de 5% en poids du poids total du noyau matriciel enrobé de la première couche d'enrobage.

2. La composition pharmaceutique selon la revendication 1, où la dite composition est une forme posologique solide telle qu'un comprimé, un sachet, ou des mini comprimés dans une capsule.

3. La composition pharmaceutique selon la revendication 1 ou 2, comprenant en outre des liants, des agents de remplissage, des agents de glissement ou des lubrifiants supplémentaires.

4. Un procédé pour la préparation d'une forme posologique solide à libération modifiée de la Toltérodine ou un sel pharmaceutiquement acceptable de celle-ci, comme principe actif, pour administration orale consistant en un noyau matriciel comprenant le principe actif avec un mélange de polymère insoluble, de polymère soluble dans l'eau et un agent mouillant, une première couche d'enrobage sur le noyau qui comprend l'éthylcellulose et est de moins de 5% en poids du poids du noyau sans enrobage et une seconde couche d'enrobage sur la première couche d'enrobage qui comprend Kollicoat MAE 30DP et le 1,2-propylène glycol et est de moins de 5% en poids du poids total du noyau matriciel revêtu (enrobé), le dit procédé comprenant :
- dissoudre la quantité totale de l'agent mouillant;
- former un mélange homogène en mélangeant la quantité totale de Toltérodine ou un sel pharmaceutiquement acceptable de celle-ci avec la quantité totale de polymère insoluble dans l'eau;
- malaxer le mélange ci-dessus avec la solution d'agent de mouillage;
- ajouter à la solution formée, la quantité totale du polymère soluble dans l'eau et la quantité totale d'au moins un excipient optionnel comme un diluant, un liant, un désintégrant, un agent de glissement, un lubrifiant et granuler par voie humide jusqu'à l'obtention d'uniformité;
- sécher la masse mouillée;
- tamiser la masse séchée et ajouter au mélange tamisé les quantités totales d'au moins un excipient supplémentaire comme un liant, un diluant, un désintégrant, un lubrifiant et/ou un agent de glissement et mélanger jusqu'à l'obtention d'uniformité;
- formuler le mélange résultant en forme posologique solide en compressant sous la forme désirée de comprimé ou mini comprimés;
- appliquer la première couche d'enrobage, sur le noyau, et comprenant l'éthylcellulose;
- appliquer la seconde couche d'enrobage au-dessus de la première couche d'enrobage, comprenant Kollicoat MAE 30DP et le 1,2-propylène glycol; et
- dans le cas de min-comprimés, remplir les mini-comprimés dans des capsules rigides de gélatine.
